# NEW EUROPEAN PATENT SPECIFICATION

(11) **EP 1 064 275 B2**
(45) Date of publication and mention of the opposition decision: **19.04.2006**
(45) Mention of the grant of the patent: 06.11.2002
(21) Application number: 99909407.1
(22) Date of filing: 05.03.1999
(51) Int. Cl.: C07D 309/30, C07D 319/06, C07D 235/30

(54) **PROCESS FOR PRODUCING SIMVASTATIN AND/OR ITS DERIVATIVES**
VERFAHREN ZU ERZEUGUNG VON SIMVASTATIN UND/ODER DEREN DERIVATEN
PROCEDE DE PRODUCTION DE SIMVASTATINE ET/OU DE SES DERIVES

(30) Priority: 05.03.1998 NL 1008502; 27.05.1998 EP 98201762
(43) Date of publication of application: 03.01.2001
(73) Proprietor: Synthon B.V., 6545 CM Nijmegen (NL)
(72) Inventor: VAN DALEN, Frans, NL-5673 BG Nuenen (NL); LEMMENS, Jacobus, Maria, NL-6585 KM Mook (NL); VAN HELVOIRT, Gertruda, Antonetta, Philomina, NL-6546 KM Nijmegen (NL); PETERS, Theodorus, Hendricus, Antonius, NL-6814 JB Arnhem (NL); PICHA, Frantisek, 615 00 Brno (CZ)
(74) Representative: Duxbury, Stephen
(86) International application number: PCT/NL1999/000119
(87) International publication number: WO 1999/045003

(56) References cited:
- EP-A- 0 299 656
- EP-A- 0 864 569
- WO-A-98/32751
- GB-B- 648 886
- US-A- 5 393 893
- US-A- 5 763 646
- Greene, "Protective Groups in Organic Synthesis", 1981, pages 10-14,21-25,72-86,293-298
- Carey et al, "Advanced Organic Chemistry", 1977, pages 408-414
- Carey et al. "Advanced Organic Chemistry, Part B: Reactions and Synthesis", 1990, pages 677-685
- J.Am.Chem.Soc. 1972, 94, 6190-6191
- Morrison et al, "Organic Chemistry", 11th edition, 1977, pages 559,729
- Jones, "Organic Chemistry", 1997, page 1077
- J.Am.Chem.Soc. 1950. 72, 5635
- McOmie, "Protective Groups in Organic Synthesis", 1973, pages 95-108

## Description

The present invention relates to a process for producing simvastatin and/or derivatives thereof as well as to a process for producing intermediates for said compounds, and to various intermediates themselves.

Certain hexahydronaphtalene derivatives are known as potent inhibitors of the enzyme HMG-CoA reductase, the rate-controlling enzyme in the biosynthetic pathway for formation of cholesterol in the human body. Well known examples of these compounds are mevastatin (US-A-3983140), lovastatin (US-A-4231938), pravastatin (US-A-4346227) or simvastatin (US-A-4444784). All of these compounds are important pharmaceuticals and are widely used in hyperchotesterolaemic treatments.

Mevastatin, lovastatin and pravastatin are natural fermentation products which possess a 2-methylbutyrate side chain in the 8- position of their hexahydronaphtalene ring system; it has been proven that products possessing a 2,2 dimethylbutyrate side chain in the same position (e.g. simvastatin (formula (A)) are even more active. Simvastatin is however, not naturally occurring.

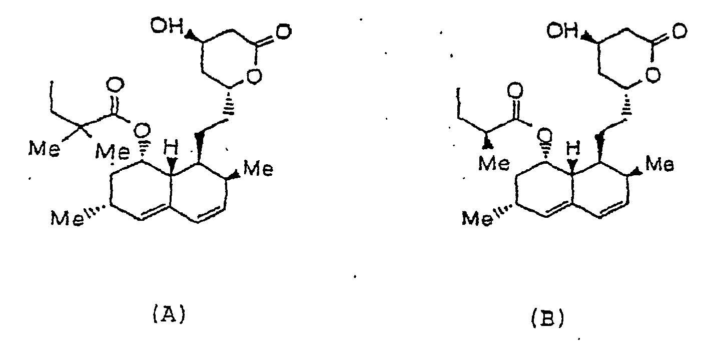

One route to introduce an additional α-methyl group to the 8-acyl side chain of lovastatin (formula (B)) or its analogues is disclosed in US-A-4,444,784. This process involves indirect methylation of the said side chain through several chemical steps: deesterification of the whole 2-methylbutyrate side chain, protection of the 4-hydroxy group in the pyranone ring by a tert-butyldimethylsilyl protective group, reesterification of the protected lactone with 2,2-dimethylbutyric acid, and deprotection of the hydroxy group of the pyranone ring. This procedure involves multiple chemical reactions with a low overall yield.

Another route, based on direct methylation of the 8-acyl side chain of lovastatin and its analogues is disclosed in US-A-4,582,915. Direct methylation of the 2-methylbutyrate side chain of lovastatin is achieved, after conversion to an alkali metal salt thereof, using a methylhalide in the presence of a strong base (metal alkylamide). Such a process exhibits disadvantages including low conversion; resulting in contamination of the product-by a significant concentration of unconverted starting material and relatively high concentration of by-products.

The problems of low yields and poor quality of the final product have been addressed in a process disclosed in US-A-4,820,850. This procedure comprises:
a) treatment of lovastatin with butylamine followed to achieve ring-opening of the lactone, followed by the protection of hydroxyl-groups therein with tert-butyldimethylsilyl chloride;
b) treatment of the obtained intermediate with an alkalimetal amide followed by contact with alkylhalide to add an alkyl group to the 2-position of butyrate side chain;
c) removal of the silyl protective groups by an acid, preferably being hydrofluoric acid;
d) treatment with dilute base to hydrolyse the alkylamide; and
e) heating of the resulting carboxylate salt in a hydrocarbon solvent.to form the lactone.

Another direct methylation process is described in US-A-5,393,893. Here, a lovastatin-C₃-C₇-alkyl amide, cycloalkylamide or aralkylamide is prepared, the hydroxyl-groups thereof are protected with a phenylboronic acid and the resulting intermediate is further reacted with an alkylhalide in the presence of a base to introduce the alkyl moiety into the butyrate side chain. The subsequent steps leading to simvastatin involve, similarly as in the preceding patent, the removal of the protective groups, hydrolysis of the alkylamide and relactonization to form simvastatin.

As apparent, the above synthetic routes, which involve the step of direct methylation, differ from each other namely by the nature of OH-protective groups in the reaction intermediates. These protected intermediates can be characterized by the presence of a C-O-Si- or C-O-B- linkage in their molecules.

However, in these known routes, the intermediates are quite unstable towards environmental hydrolysis and unstable towards strongly alkaline conditions during the methylation. As a result, undesirable amounts of by-products are formed during the synthesis. To obtain a product having the desired pharmaceutical quality, these by-products have to be removed by additional purification methods which lowers the overall yield and increase cost. Furthermore, the protecting agents used are economically undesirable.

An object of the present invention is to provide an alternative, improved, economic process for the synthesis of simvastatin and its analogues.

The present invention in a first aspect relates to the use of ether-based hydroxyl protecting groups in the synthesis of simvastatin and its analogues.

The invention provides process for producing simvastatin and/or derivatives thereof according to claim 1, and a process for preparation of a compound of formula (III) according to claim 4.

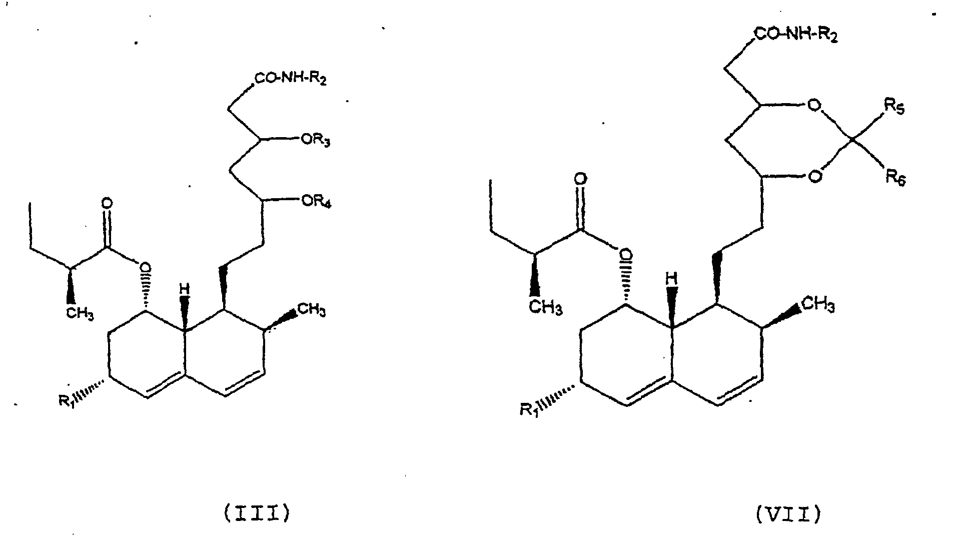

(The process involving the intermediates VII and VIII, and the compounds of formula VII and VII are not part of the invention as claimed).

The compounds of formula (III) and (VII) can be subjected to direct alkylation with an alkylating agent of formula R₇X to produce compounds of formula (IV) and (VIII), respectively.

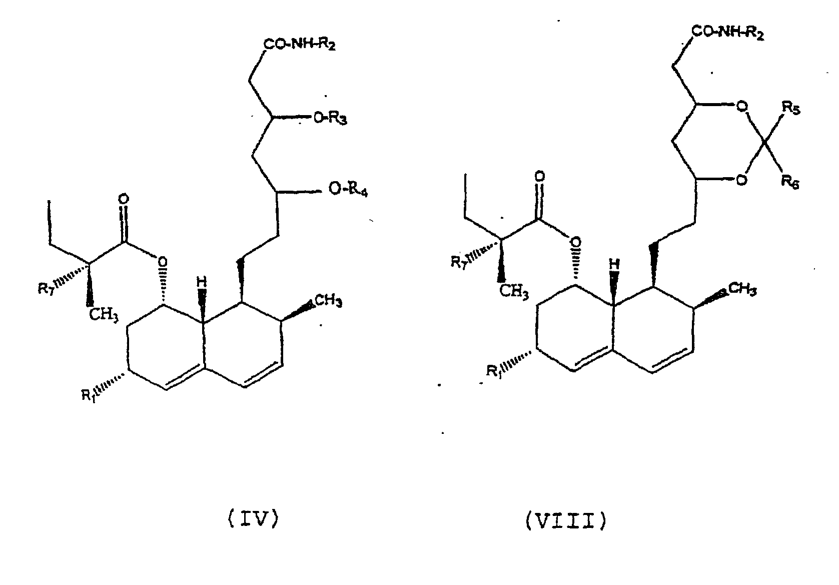

R⁷ is methyl or ethyl, X represents a leaving group, preferably a halide, most preferably chlorine, bromine and iodine and R³ - R⁶ are defined above. The compounds of formula (III) and (IV) are a second embodiment of the invention.

Simvastatin or its analogue can be subsequently produced by deprotecting the compound of formula (IV) or (VIII), hydrolyzing the alkylamide, and reforming the lactone ring to produce a compound of formula (VI).

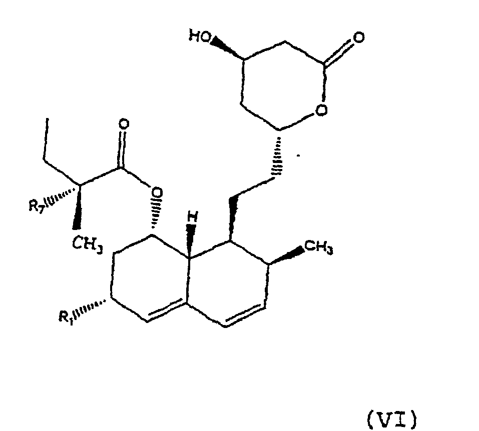

By using the R₃ and R₄ protecting groups, the present invention can provide an economical, convenient and efficient process for making compounds of formula (VI) in high purity.

The following reaction scheme summarizes various aspects and embodiments of the present invention.

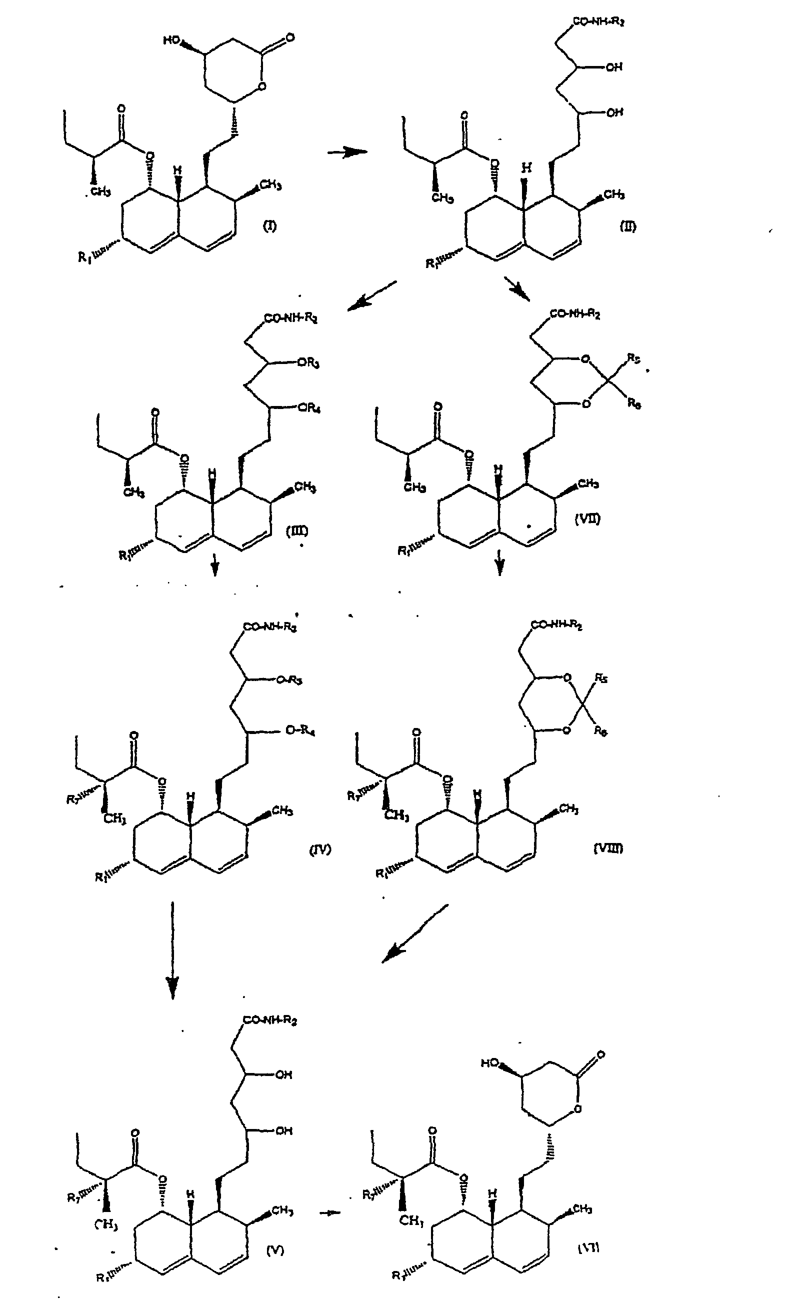

The process involving intermediates VII and VIII, and the compounds VII and VIII are not part of the invention as claimed.

The compounds of formula (I) are known, naturally occurring compounds. R¹ is hydrogen or methyl. The compounds of formula (II) are formed by carrying out a ring opening reaction with an amine of the formula R²NH₂. R² represents a straight or branched alkyl group of 1 to 8 carbon atoms, a cycloalkyl group of 3 to 7 carbon atoms, or an aralkyl group having 1 to 6 carbon atoms in the alkyl chain. Typically, the aralkyl group contains 1 to 4 carbon atoms in the alkyl moiety although such is not required, and the aromatic moiety is phenyl or naphthyl. Examples of suitable R² groups include methyl, ethyl, propyl (iso- and n-forms), butyl (tert-, iso- and n-forms), cyclohexyl, cyclopentyl, benzyl, phenethyl, and 3-phenylpropyl.

When using a low boiling amine (such as methylamine or ethylamine) to form the alkylamide of formula (II), it is preferred that the reaction is carried out in an inert solvent, such as tetrahydrofuran or toluene. When employing high boiling amines (e.g. butylamine), the amine itself can be used as the solvent. After evaporation of the solvent and/or removal of the unreacted amine, the compound (II) is obtained. While the use of alkylamines having as few as three carbon atoms has been described in US-A-4820850 and US-A-5393893, the present invention further specifically contemplates the use of C₁ and C₂ amines (methylamine, ethylamine) in the amidation reaction as described above. This embodiment allows for easy purification of the product compound of formula (II) vis-a-vis the amine reactant in that any unused amine reactant can be readily volatized off. This provides a convenient and effective method to remove any excess amine impurity, especially if the compound of formula (II) is not isolated before the next reaction step.

The hydroxyl group of the amide of formula (II) are then protected with carbon-terminated protective groups to form an ether as shown in formulas (III) and (VIII). R³ and R⁴ independently represent a dioxanyl, tetrahydrofuranyl, tetrahydropyran-2-yl, tetrahydrothiopyran-2-yl, 4-methoxytetrahydropyran-2-yl, 1,4 dioxan-2-yl or tetrahydrofuran-2-yl.

The R³ and R⁴ groups can be formed by reacting the amide of formula (II) with an appropriate donor of the carbon-terminated protective group by generally known methods. For example, by a substitution reaction with appropriate alkyl or benzyl hafogenides or sulphates in the presence of a base, or by an addition reaction with an appropriate unsaturated compound (e.g. with 3,4-dihydro 2H-pyrane) in a suitable solvent, e.g. in dichloromethane, under catalysis by an acid (e.g. p-toluenesulfonic acid). Usually only one type of donor compound is used and thus R³ and R⁴ are usually identical to one another.

In formula (VIII, R⁵ and R⁶ each independently represent hydrogen, an alkyl group, an aryl group, an aralkyl group, an alkoxy group, or an ether group. These groups have the same meaning as defined for R³ and R⁴ wherein the "alkoxy group" is an "alkyl group" linked by an oxygen atom to the ring carbon. Examples of R⁵ and R⁶ include hydrogen, lower alkyl (methyl, ethyl, propyl, butyl, etc.), phenyl, benzyl, methoxy, ethoxy or methoxymethyl.

The "cyclic ethers" of the formula (VII) can be made by reacting the amide of formula (II) with an appropriate donor or the carbon-terminated protective group by generally known methods. Typically, were at least one of groups R⁵ and R⁶ is hydrogen, the reaction uses a donor having an activated C=O group of the appropriate aldehyde (e.g. benzaldehyde). When neither R⁵ nor R⁶ is hydrogen, such as when both are alkyl, e.g. methyl, the compounds can be formed by the reaction with an activated ketone. For example, acetone or its alpha substituted derivatives can be used in the presence of a strong acid (preferably p-toluenesulfonic acid) and a dehydrating agent (preferably silica gel, molecular sieves, sodium sulfate, or copper (II) sulfate), preferably at room temperature.

Alternatively, 2,2-dimethoxypropane is another example of a donor compound that can be used in the presence of a strong acid (preferably p-toluenesulfonic acid), preferably at room temperature.

Thus, the "cyclic-ethers" of the formula (VII) can also be regarded as being cyclic acetals or ketals.

The reaction with a donor of the protective group generally proceeds with almost 100% conversion (e.g. 99.7% in case of acetonides) and under mild reaction conditions.

The following compounds of the formula (III) and (VII) are examples of useful compounds of the present invention (compounds of formula VII are not part of the invention as claimed):
lovastatin ethylamide bis-tetrahydropyran-2-ylether (compound (III), wherein R¹=methyl, R²=ethyl, R³=R⁴=tetrahydropyran-2-yl)
lovastatin n-butylamide bis-tetrahydropyran-2-ylether (compound (III), wherein R¹=methyl, R²=n-butyl, R³=R⁴=tetrahydropyran-2-yl)
lovastatin ethylamide acetonide (compound (VII) wherein R¹=methyl, R²=ethyl, R⁵=R⁶=methyl
lovastatin butylamide acetonide (compound (VII) wherein R¹=methyl, R²=n-butyl, R⁵=R⁶=methyl

Subsequently, the compounds of formula (IV) or (VIII) are prepared from compounds (III) or (VII) by alkylation. The alkylation of the protected amide (III) or (VII) can be carried out according to known methods, such as by adding an alkylating agent of formula R₇X in the presence of a base. For example, the amide can be firstly treated with an alkali metal amide (which is prepared by known methods by a combination of n-butyllithium with a lower secondary amine, e.g. pyrrolidine or piperidine, in an etheral solvent, e.g. tetrahydrofuran) at a low temperature (-30°C to -40°C) and then the alkylating agent an alkylhalide of the general formula R⁷-X wherein R⁷ represents methyl or ethyl and X represents a leaving group such as chlorine, bromine or iodine (e.g. methyliodide) can be added, preferably at the same temperature. The alkylation reaction is preferably performed in inert atmosphere, e.g. under nitrogen. The reaction mixture can then be treated with cold water and the desired compound (IV) of (VIII) isolated. If the desired product is a oil, it can be isolated by extraction into an organic solvent, preferably ethyl acetate, followed by evaporation of the said solvent. If it is a solid, it can be isolated in the solid state after extraction and evaporation steps, namely by trituration of the residue after evaporation with an appropriate solvent in which the product is not soluble, e.g. in hexane.

The following compounds of the formula (IV) and (VIII) are examples of the compounds of the present invention (compounds of formula VIII are not part of the invention as claimed):
Simvastatin ethylamide bis-tetrahydropyran-2-ylether (compound (IV), wherein R¹=methyl, R²=ethyl, R³=R⁴=tetrahydropyran-2-yl, R⁷=methyl)
Simvastatin n-butylamide bis-tetrahydropyran-2-ylether (compound (IV), wherein R¹=methyl, R²=n-butyl, R³=R⁴-tetrahydropyran-2-yl, R⁷=methyl)
Simvastatin ethylamide acetonide (compound VIII) wherein R¹=methyl, R²=ethyl, R⁵=R⁶=R⁷methyl)
Simvastatin butylamide acetonide (compound VIII) wherein R¹=methyl, R²=n butyl, R⁵=R⁶=R⁷=methyl

The next step achieves deprotection of the hydroxyl groups in compounds (IV) or (VIII) to form a compound of formula (V). For this purpose, the ether linkages in compounds (IV) or (VIII) are subjected to hydrolysis, preferably to acidic hydrolysis, preferably using an aqueous solution of a strong organic acid e.g. methane sulfonic acid in a mixture with water miscible alcohol, e.g. methanol. Deprotected alkylated amide (V) results both from the compound (IV) and from the compound (VIII).

Similarly, a strong acidic resin can also be used for the same purpose or specific known cleavage methods can be applied.

In the last step, hydrolysis of the amide group in the compound (V) can be performed without need of its isolation, by preferably heating the reaction mixture with an excess of an aqueous solution of a strong inorganic base (e.g. sodium hydroxide) under continuous removal of the alcohol. The reaction mixture is extracted by a water immiscible-solvent, e.g. ethyl acetate, the organic layer is evaporated and final lactonization to yield the compound (VI) is performed by conventional methods, i.e. by boiling the distillation rest with toluene under continuous removal of water.

In all the steps, the solutions of any intermediate may be purified by treatment of activated charcoal, silica gel, kieselguhr or other suitable materials; another suitable method of purification is crystallization from a proper solvent. Nevertheless, these purifications must be applied only for characterization purposes. Due to high conversion and low amount of side products, all the reaction steps of the present invention, i.e. the process involving the intermediates III and IV, proceed in practical production substantially without the need of purification. All the reaction steps are preferably performed under nitrogen atmosphere.

This process involves OH-protected derivatives of lovastatin or mevastatin as reaction intermediates, said derivatives having good stability, being easily synthetizable and allows preparation of the desired compounds in good yield and having good purity, wherein the use of expensive and unstable reactants for protection of the hydroxyl groups is substantially obviated.

The compounds of the present invention are characterized by infra-red spectra, NMR spectra and mass spectra as exemplified in the example, see later.

In some cases, two new centers of chirality (e.g. on the carbons 2' in tetrahydropyrane rings) are introduced, so that certain compounds of the present invention exist as a mixture of enantiomers. It cannot be excluded that a person skilled in the art may easily find a method of their resolution into optically pure isomers, if necessary.

The compounds of the present invention can exist in unsolvated as well as solvated forms, including hydrated forms. In general, the solvated forms are equivalent to the unsolvated forms for purpose of the invention.

Except where specifically defined to the contrary, the term alkyl includes both the straight chain, branched chain and/or cyclic chain species with the same number of carbon atoms.

The following examples illustrate the invention.

### EXAMPLES

### Example 1a

Preparation of lovastatinethylamide: N-ethyl-7-[1(S),2(S),6(R),7,8(S),8a(R)-hexahydro-2,6-dimethyl-8-g2 (S)-methylbuanoyl]oxy]-1-naphthyl]-3(R),5(R)-dihydroxyheptanamide, compound of formula (II) (wherein R¹ is methyl and R² is ethyl).

A mixture of lovastatin (5.0 g, 0.012 mol) and 2N solution of ethylamine in tetrahydrofuran (37 ml, 0,074 mol) was heated to gentle reflux at 70°C for 10 hours. The solution was cooled to ambient temperature and ethyl acetate (100 ml) was added; the mixture was washed with 2N hydrochloric acid (3x50 ml), The combined aqueous layers were washed with ethyl acetate (100 ml) and the combined organic layers with water (2x100 ml). The organic layer was dried over sodium sulphate, filtered and evaporated at reduced pressure to give an orange oil.

NMR (CDCl₃, delta scale): 6.22 (NH), 5.98 (C_{4'}-H), 5.78 (C_{3'}-H), 5.51 (C_{5'}-H), 5.41 (C_{8'}-H), 4.81 (OH), 4.21 (C₃-H), 3,79 (C₅-H and OH) and 3.29 (C_{1"}-H).

Mass spectrum: m/e 449 (M⁺) with major fragments at m/e 431, 347, 224, 198, 172 and 159.

Infra red spectrum (NaCl): principal peaks at wave numbers (cm⁻¹) 3200-3500 (OH and NH), 2900-3015 (C-H), 1753 (ester C=O), 1664 (amide C=O), 1559 (amide C=O), 1208 (ester C-O-C).

### Example 1b

Preparation of lovastatin ethylamide bis-tetrahydropyran-2-yl ether: N-ethyl-7-[1 (S),2(S),6(R),7,8(S),8a(R)-hexahydro-2,6-dimethyl-8-[[2(S)-methylbutanoyl]oxy]-1-naphthyl]-3(R),5(R)-bis[(tetrahydropyranyl)oxy]heptanamide, compound of formula (III) (wherein R¹ is methyl, R² is ethyl, R³ and R⁴ are tetrahydropyran-2-yl).

To a solution of lovastatin ethylamide of the Example 1a (1.1 g, 2.5 mmol) in dichloromethane (10 ml), 3,4-dihydro-2H-pyran (1.1 ml, 12.1 mmol) and 24 mg paratoluenesulfonic acid monohydrate (24 mg, 0,13 mmol) were added. The solution was stirred at 20° -15°C for 1,5 hours. Then cyclohexane (20 ml) and 5% mN aqueous sodium bicarbonate (20 ml) was added. The mixture was stirred vigorously and the layers were allowed to separate. The organic layer was washed successively with 5% m/V aqueous sodium bicarbonate (20 ml) and distilled water (20 ml). The organic layer was dried oversodium sulfate, filtered and evaporated to dryness under reduced pressure to obtain an orange coloured oil.

NMR (CDCl₃, delta scale): 6.92, 6.77, 6.39, 6.22 (NH), 5.98 (C_{4'}-H), 5.79 (C_{3'}-H), 5.51 (C_{5'}-H), 5.35 (C_{8'}-H).

Mass spectrum: m/e 617 (M⁺) with major fragments at m/e 516, 449, 347, 198, 172, 159 and 85.

Infra red spectrum (NaCl): principal peaks at wave numbers (cm⁻¹) 3324 (NH), 2880-3025 (C-H), 1730 (ester C=O), 1660 (amide C=O), 1540 (amide C=O), 1190 (ester C OC).

### Example 1c

Preparation of Simvastatinethylamide bistetrahydropyran-2-yl ether: N-ethyl-7-[1 (S), 2(S), 6(R), 7,8(S), 8a (R)-hexahydro-2,6-dimethyl-8-[[2,2-dimethylbutanoyl]oxy]-1-naphthyl]-3(R),5(R)-bis [(tetrahydropyranyl)oxy]heptanamide, compound of formula (IV) (wherein R¹ is methyl, R² is ethyl, R³ is tetrahydropyran-2-yl and R⁷ is methyl).

n-Butyllithium in hexane (26 ml, 0,041 mol) was added to a stirred solution of pyrrolidine (3.5 ml 0.042 mol) in anhydrous, tetrahydrofuran (12 ml) at -20 °C under an inert atmosphere of nitrogen. The mixture was stirred at -20 °C for 30 minutes and then added dropwise to a stirred solution of lovastatinethylamidetetrahydropyranyl ether of the Example 1b (11.0 g, 0,018 mol) in tetrahydrofuran (48 ml) precooled to -35°C, in such a rate as to keep the temperature between -30°C and -35°C. (see Note) After completion of the addition the mixture was stirred at -35°C for 2 hours. Then methyliodide (1,7 ml, 0,027 mol) was added in one portion. After an initial 15°C exotherm, the solution was recooled to -30°C and maintained for an additional 30 minutes. The mixture was then quenched by addition of water (80 ml) and the mixture was allowed to warm to -5°C, while stirring. The phases were separated and the aqueous layer was extracted with ethyl acetate (2x50 ml).

The combined organic layers were extracted with 1N aqueous hydrochloric acid (100 ml). The resulting organic phase was concentrated at reduced pressure to obtain an orange coloured oil.

NMR (CDCl₃, delta scale): 6.92, 6.78, 6.42, 6.25 (NH), 5.98 (C_{4'}-H), 5.78 (C_{3'}-H), 5.49 (C_{5'}-H), 5.33 (C_{8'}-H)

Infra red spectrum (NaCl): principal peaks at wave numbers (cm⁻¹) 3338 (NH), 2865-3000 (C-H), 1716 (ester C=O), 1649 (amide C=O), 1540 (amide C=O), 1162 (ester C-OC).
Note: The addition order can also be reversed without any influence on the results.

### Example 1d

Preparation of Simvastatin, compound of the formula (VI) (wherein R¹ is methyl and R⁷ is methyl).

### Step i:

Preparation of Simvastatinethylamide: N-ethyl-7-[1(S),2(S),6(R),7,8(S),8a(R)-hexahydro-2,6-dimethyl-8-[[2,2-dimethylbutanoyl]oxy]-1-naphthyl]-3(R),5(R)-dihydroxyheptanamide, compound of formula (V) wherein R¹ is methyl, R² is ethyl and R⁷ is methyl).

Methanol (50 ml) was added to the crude Simvastatin ethylamide bistetrahydropyran-2-yl ether of the Example 1c (11.2 g). Then water (4 ml) and methanesulfonic acid (125 µl) were added and the resulting solution was stirred for 6 hours at 30°C.

For characterization purpose, an aliquot was evaporated, dissolved in ethyl acetate and extracted with an aqueous solution of sodium bicarbonate, water, dried over sodium sulfate, filtrated and evaporated to dryness to obtain an orange coloured oil.

NMR (CDCl₃, delta scale): 6.29 (NH), 5.98 (C_{4'}-H), 5.78 (C_{3'}-H), 5.49 (C_{5'}-H), 5.40 (C_{8'}-H), 4.86 (OH), 4.21 (C₃-H), 3.86 (OH), 3.79 (C₅-H), and 3.29 (C_{1"}-H).

Infra red spectrum (NaCl): principal peaks at wave numbers (cm⁻¹) 3297-3608 (OH and NH), 2878-3040 (C-H), 1716 (ester C=O), 1635 (amide C=O).

### Step ii:

### Preparation of Simvastatin

To the clear methanol solution from the preceded step there was added 2N NaOH aqueous solution (60 ml) and the solution was heated to reflux for 5 hours while distillate (70 ml) was collected at ambient pressure. The mixture was allowed to cool down to ambient temperature, diluted with water (15 ml) and cooled to 10°C while adjusting the pH to 3 with 5N HCl (35 ml).

Ethyl acetate (100 ml) was added and after agitation, the phases were allowed to settle and were separated. The aqueous layer was extracted with ethyl acetate (2x50 ml), the combined organic layers were dried over sodium sulfate, filtered and evaporated at reduced pressure.

Then toluene was added (60 ml) and the solution was allowed to reflux with continuous separation of water for 6 hours.

Evaporation of the toluene, crystallization from cyclohexane and recrystallization from methanol/water yielded analytically pure Simvastatin.

Melting point: 131° - 133 °C.

[α]_{D} (5 mg/ml acetonitrile) : +286°

NMR (CDCl₃, delta scale): 5.99 (C₅-H), 5.79 (C₆-H), 5.51 (C₄-H), 5.37 (C₁-H), 4.63 (C_{2'}-H) and 4.36 (C_{4'}-H).

Mass spectrum: m/e 418 (M⁺) with major fragments at m/e 302, 284, 199, 173 and 159.

Infra red spectrum (KBr): principal peaks at wave numbers (cm⁻¹) 3545 (OH), 2850-3050 (C-H), 1715 (ester C=O), 1700 (amide C=O), 1275 (lactone C-O-C) and 1170 (ester C-O-C).

### Examples 2 to 5

Following the procedure substantially as described in Examples 1a to 1d(i), the following compounds (II) to (V) were prepared:

| Example | formula | R¹ | R² | R³=R⁴ | R⁷ |
|---|---|---|---|---|---|
| 2 | II | methyl | n-butyl | - | - |
| 3 | III | methyl | n-butyl | THP | - |
| 4 | IV | methyl | n-butyl | THP | methyl |
| 5 | V | methyl | n-butyl | - | methyl |
| (THP = tetrahydropyran-2-yl) | | | | | |

### Example 2

### Example 2 = N-butyl-7-[1 (S),2(S),6(R),7,8(S),8a(R)-hexahydro-2,6-dimethyl-8-[[2(S)-methylbutanoyl]oxy]-1-naphthyl]-3(R),5(R)-dihydroxyheptanamide,compound of formula (II):

Melting point: 76°C

NMR (CDCl₃, delta scale): 6.10 (NH), 5.98 (C_{4'}-H), 5.79 (C_{3'}-H), 5.2 (C_{5'}-H), 5.42 (C_{8'}-H), 4.76 (OH), 4.20 (C₃-H), 3.80 (C₅-H), 3.62 (OH) and 3.25 (C_{1"}-H).

Mass spectrum: m/e 477 (M⁺) with major fragments at m/e 459, 441, 375, 360, 339, 342, 251, 199, 173 and 159.

Infra red spectrum (KBr): principal peaks at wave numbers (cm⁻¹) 3200-3520 (OH and NH), 2885-3030 (C-H), 1730 (ester C=O), 1635 (amide C=O), 1605 (amide C=O) and 1185 (ester C-O-C).

### Example 3

### Example 3 = N-butyl-7-[1 (S),2(S),6(R),7,8(S),8a(R)-hexahydro-2,6-dimethyl-8-[[2(S)-methylbutanoyl]oxy]-1-naphthyl]-3(R),5(R)-bis[(tetrahydropyranyl)oxy]heptanamide, compound of formula (III):

Melting point: 131°-133°C.

[α]_{D} (5 mg/ml acetonitrile) : +286°

NMR (CDCl₃, delta scale): 5.99 (C₅-H), 5.79 (C₆-H), 5.51 (C₄-H) 5.37 (C₁-H) 4.63 (C_{2'}-H) and 4.36 (C_{4'}-H).

Mass spectrum: m/e 418 (M⁺) with major fragments at m/e 302, 284, 199, 173 and 159.

Infra red spectrum (KBr): principal peaks at wave numbers (cm⁻¹) 3545 (OH), 2850-3050 (C-H), 1715 (ester C=O), 1700 (amide C=O), 1275 (lactone C-O-C) and 1170 (ester C-O-C).

### Example 4

### Example 4 = N-butyl-7-[1 (S),2(S),6(R),7,8(S),8a(R)-hexahydro-2,6-dimethyl-8-[[2,2-dimethylbutanoyl]oxy]-1-naphthyl]3(R),5(R)bis[(tetrahydropyranyl)oxy]heptanamide, compound of formula (IV)

NMR (CDCl₃, delta scale): 6.95, 6.81, 6.42, 6.26 (NH), 5.98 (C_{4'}-H), 5.78 (C_{3'}-H), 5.49 (C_{5'}-H) , and 5.33 (C_{8'}-H).

Infra red spectrum (NaCl): principal peaks at wave numbers (cm⁻¹) 3365 (NH), 2878-3030 (C-H), 1730 (ester C=O) and 1662 (amide C=O).

### Example 5

### Example 5 = N-butyl-7-[1(S),2(S),6(R),7,8(S),8a(R)-hexahydro-2,6-dimethyl-8-[[2,2-dimethylbutanoyl]oxy]-1-naphthyl]-3(R),5(R)-dihydroxyheptanamide, compound of formula (V) example 1d;

NMR (CDCl₃, delta scale): 6.42 (NH), 5.98 (C_{4'}-H), 5.78 (C_{3'}-H), 5.50 (C_{5'}-H), 5.42 (C_{8'}-H), 4.81 (OH), 4.19 (C₃-H), 3.79 (C₅-H), 3.74 (OH) and 3.25 (C_{1"}-H).

Mass spectrum: m/e 477 (M⁺) with major fragments at m/e 459, 441, 375, 360, 339, 342, 251 , 199, 173 and 159.

Infra red spectrum (NaCl): principal peaks at wave numbers (cm⁻¹) 3220-3520 (OH and NH), 2885-3030 (C-H), 1720 (ester C=O), 1645 (amide C=O), 1560 (amide C=O) and 1160 (ester C-O-C).

### Example 6 (not part of the invention as claimed)

Preparation of lovastatin ethylamide acetonide: N-ethyl-7-[1 (S),2(S),6(R),7,8(S),8a(R)-hexahydro-2,6-dimethyl-8-[[2(S)-methylbutanoyl]oxy]-1-naphthyl]-3(R),5(R)-O-isopropylideen] heptanamide, compound of formula (VII) (wherein R¹ is methyl, R² is ethyl and R⁵=R⁶ is methyl).

To a solution of lovastatinethylamide of the Example 1a (3.0 g, 6.7 mmol) in 2,2-dimethoxypropane (20 ml), 210 mg para-toluenesulfonic acid monohydrate (210 mg, 1.1 mmol) was added. The solution was stirred at 20°C-25°C for 30 minutes. Then ethyl acetate (10 ml) and 5% m/V aqueous sodiumbicarbonate (20 ml) was added. The mixture was stirred vigorously and the layers were allowed to separate. The organic layer was washed successively with 5% m/V aqueous sodiumbicarbonate (20 ml) and brine (20 ml). The organic layer was dried over sodiumsulfate, filtered and evaporated to dryness under reduced pressure to obtain a yellow coloured oil. For characterization purposes, an aliquot was crystallized from n-hexane to obtain an off white solid.

NMR (CDCl₃, delta scale): 6.26 (NH), 5.99 (C_{4'}-H), 5.78 (C_{3'}-H), 5.51 (C_{5'}-H), 5.34 (C_{8'}-H), 1.44, 1.38 (O-C (CH₃)-CH₃).

Infra red spectrum (KBr): principal peaks at wave numbers (cm⁻¹) 3300 (NH), 2870-3010 (C-H), 1728 (ester C=O), 1657 (amide C=O), 1542 (amide C=O).

Melting point:90-91 °C

### Example 7 (not part of the invention as claimed)

Preparation of Simvastatinethylamide acetonide: N-ethyl-7-[1 (S),2(S),6(R),7,8(S),8a(R)-hexahydro-2,6-dimethyl-8-[[2,2-dimethylbutanoyl]oxy]-1-naphthyl]-3(R),5(R)-O-isopropylideen] heptanamide, compound of formula (VIII) (wherein R¹ is methyl, R² is ethyl, R⁴ is methyl and R⁵=R⁶=R⁷ is methyl).

n-Butyllithium in hexanes (30 ml, 0.048 mol) was added to a stirred solution of pyrrolidine (4.0 ml 0.048 mol) in anhydrous tetrahydrofuran (15 ml) at -20°C under an inert atmosphere of nitrogen. The mixture was stirred at -20°C for 30 minutes and then added dropwise to a stirred solution of lovastatinethylamide acetonide of the example (10.0 g, 0.021 mol) in tetrahydrofuran (50 ml) cooled to -35°C, in such a rate as to keep the temperature between -30°C and -35°C. After completion of the addition the mixture was stirred at -35°C for 2 hours. Then methyliodide (2.0 ml, 0,032 mol) was added in one portion. After an initial 15 °C exotherm, the solution was recooled to -30 °C and maintained for an additional 30 minutes. The mixture was then quenched by addition of water (80 ml) and allowed to warm to -5 °C, while stirring. The phases were separated and the aqueous layer was extracted with ethyl acetate (2x50 ml). The combined organic layers were extracted with 1 N aqueous hydrochloric acid (100 ml). The resulting organic phase was concentrated at reduced pressure to obtain an orange coloured oil.

NMR (CDCl₃, delta scale): 6.28 (NH), 5.98 (G_{4'}-H), 5.77 (C_{3'}-H), 5.50 (C_{5'}-H), 5.32 (C_{8'}-H), 1.44, 1.38 (O-C (CH₃)-CH₃).

Infra red spectrum (NaCl) : principal peaks at wavenumbers (cm⁻¹) 3310 (NH), 2894-3017 (C-H), 1718 (ester C=O), 1651 (amide C=O), 1547 (amide C=O).

### Example 8 (not part of the invention as claimed)

Preparation of Simvastatinethylamide: N-ethyl-7-[1 (S),2(S),6(R),7,8(S),8a(R)-hexahydro-2,6-dimethyl-8-[[2,2-dimethylbutanoyl]oxy]-1-naphthyl]-3(R),5(R)-dihydroxyheptanamide, compound of formula (V) (wherein R¹ is methyl, R² is ethyl, R⁷ is methyl).

Methanol (50 ml) was added to the crude Simvastatinethylamide acetonide (10.3 g). Then water (4 ml) and methanesulfonic acid (125 µl) was added and the resulting solution was stirred for 6 hours at30 °C. For characterization purpose, an aliquot was evaporated, dissolved in ethyl acetate and extracted with an aqueous solution of sodium bicarbonate, water, dried over sodium sulfate, filtrated and evaporated to dryness to obtain an orange coloured oil.

Identification date of the compound was identical with these of example 1 d, step i.

## Claims

1. Process for the preparation of a compound of formula VI: 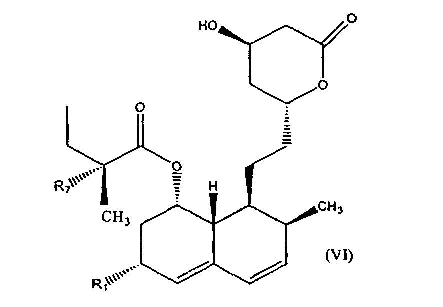 comprising the steps of:
(a) reacting a compound of the general formula I: 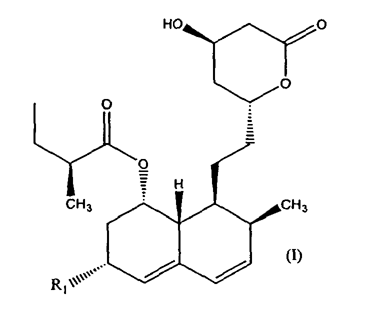 with a ring - opening agent to provide a compound having general formula II: 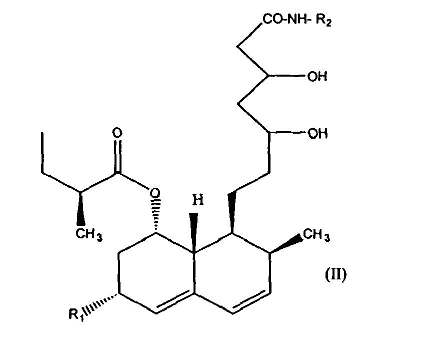 wherein:
- R₁ represents a hydrogen or a methyl group,
- R₂ represents a straight or branched alkyl chain having 1 to 8 carbon atoms, a cycloalkyl group having 3 to 7 atoms or an aralkyl group, having 1 to 6 carbon atoms in the alkyl chain and preferably represents methyl, ethyl, propyl or butyl,
(b) protecting the hydroxy groups of the thus formed compound II by reacting the compound of formula (II) with a protecting agent in order to substantially retard the reactivity thereof and provide a compound having the general formula III: 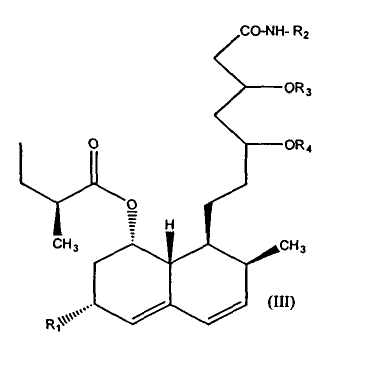 wherein the protecting agent retards the reactivity of compound II by replacing the hydrogen atom of the hydroxy groups thereof with a carbon terminated protecting group, R₃, R₄, wherein R₃, R₄ each independently represent a tetrahydropyran-2-yl tetrahydrothiopyran-2-yl, 4-methoxytetrahydropyran-2-yl a tetrahydrofuranyl group or a dioxanyl group, and
(c) reacting compound (III) with an alkylating agent of formula R₇X to obtain a compound having the formula (IV) : 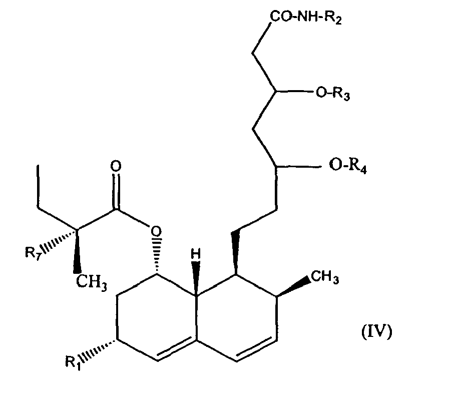 wherein:
- R₇ represents a methyl or ethyl group and X represents a leaving group, preferably a halide, most preferably chlorine, bromine or iodine,
(d) hydrolyzing the carbon terminated protecting groups R₃ and R₄ of compound (IV) to obtain a compound of formula (V) 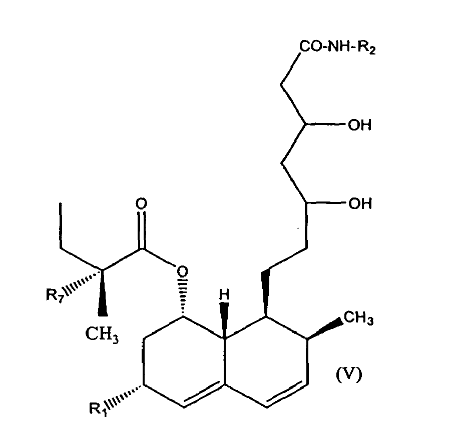 and:
(e) hydrolyzing the amidic moiety of compound (V) and relactonizing to yield the compound (VI).

2. Process according to claim 1 wherein the ring opening agent comprises an amine having the general formula R₂NH₂ wherein R₂ as defined in claim 1 represents a straight or branched alkyl chain having 1 to 8 carbon atoms, a cycloalkyl group having 3 to 7 atoms or an aralkyl group, having 1 to 6 carbon atoms in the alkyl chain and preferably represents methyl, ethyl, propyl or butyl.

3. Process according to claims 1 or 2, wherein R₃ and R₄ are the same.

4. Process for preparation of a compound of formula (III): 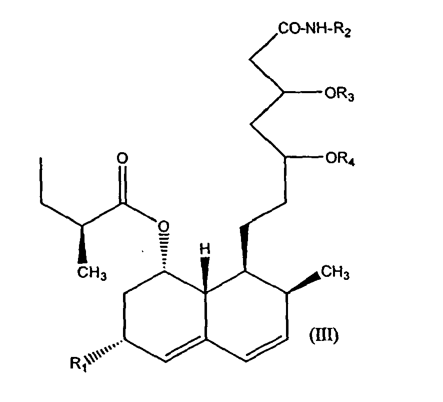 wherein:
- R₁ represents a hydrogen or a methyl group,
- R₂ represents a straight or branched alkyl chain having 1 to 8 carbon atoms, a cycloalkyl group having 3 to 7 atoms or an aralkyl group, having 1 to 6 carbon atoms in the alkyl chain and preferably represents methyl, ethyl, propyl or butyl,
- R₃ and R₄ each independently represent a tetrahydropyran-2-yl tetrahydropyran-2-yl 4-methoxytetrahydropyran-2-yl a tetrahydrofuranyl group or a dioxanyl group comprising the step of:
- reacting a compound having general formula (II) : 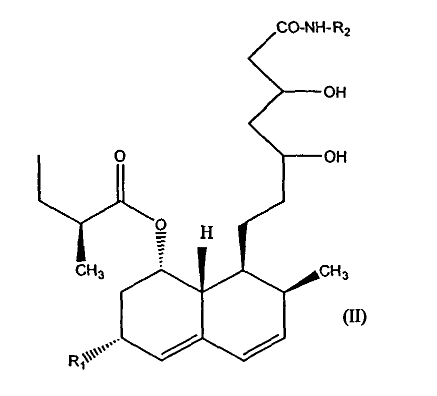
with a protecting agent to form a compound of the formula (III), wherein the hydrogen atoms of the hydroxy groups are replaced with carbon terminated protecting groups R₃ and R₄. it

5. Process according to claim 4 wherein compound II is provided by reacting a compound of the general formula (I) : 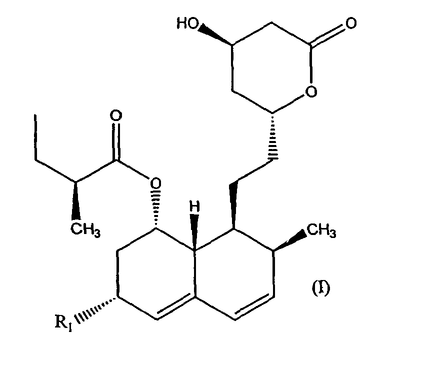 with a ring opening agent, wherein R₁ is as defined in claim 4.

6. Process according to claim 5 wherein:
the ring opening agent comprises an amine having the general formula R₂NH₂, wherein R₂ comprises a straight or branched alkyl chain having 1 - 8 carbon atoms, a cycloalkyl group having 3 - 7 atoms or an aralkyl group, having 1 to 6 carbon atoms in the alkyl chain.

7. Process according to any of the claims 5 or 6 wherein R₃ and R₄ are selected from the group consisting of 1,4 dioxan-2-yl and tertahydrofuran-2-yl.

8. Process according to any of the claims 5-7 wherein the protecting agent is a dihydropyran compound and R₃ and R₄ both represent the same tetrahydropyranyl group.

9. Process according to any of the claims 5-8, comprising the further step of alkylating compound (III) to obtain a compound having the formula (IV):

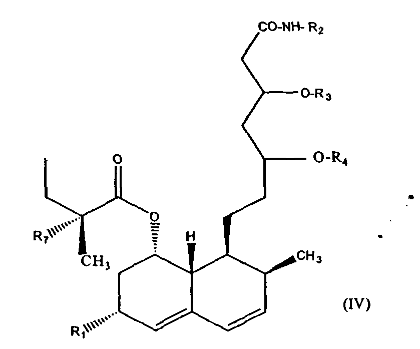

10. Process according to claim 9 wherein said alkylating step is carried out by subjecting said compound of formula (III) to a compound of the formula R₇-X, wherein R₇ represents a methyl or ethyl group and X represents a leaving group, preferably a halide, most preferably chlorine, bromine or iodine.

11. Process according to claim 10 comprising the further step of removal of the carbon terminated protecting groups from the compound (IV) whereby the compound of the formula (V): 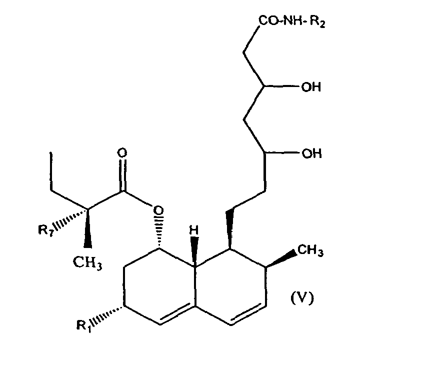 is obtained.

12. Process according to claim 11 wherein the deprotection is carried out by hydrolyzing the protecting groups.

13. Process according to claim 12, comprising the further steps of hydrolysis of the amide moiety of the compound of formula (V) and relactonization to hereby form a compound having the formula (VI).

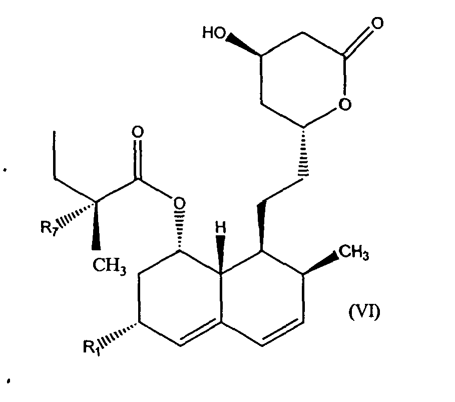

14. Compound of formula (III) or (IV), 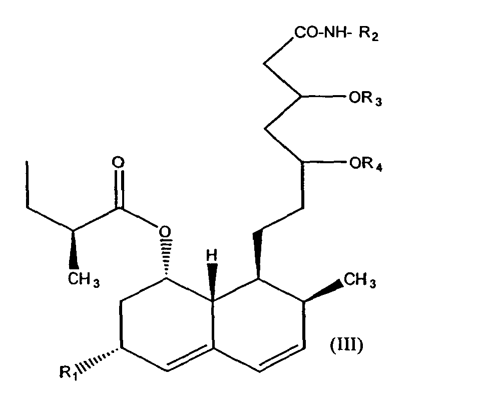 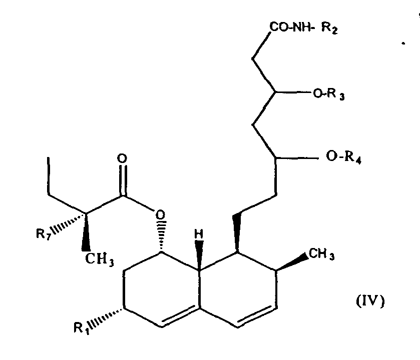 Wherein:
- R₁ is hydrogen or methyl;
- R₂ represents a straight or branched chain alkyl group having 1 to 8 carbon atoms, a cycloalkyl group having 3 to 7 carbon atoms, or an aralkyl group having 1 to 6 carbon atoms in the alkyl chain;
- R₃ and R₄ each independently represent a tetrahydropyran-2-yl tetrahydrothiopyran-2-yl 4-methoxytetrahydropyran-2-yl a tetrahydrofuranyl group or a dioxanyl group;
- and R₇ represents methyl or ethyl.

15. Compound of formula II: 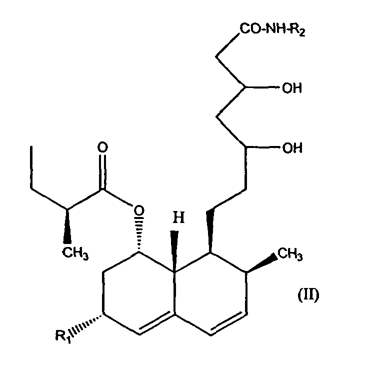 wherein R₁ is hydrogen or a methyl group and R₂ is a methyl or ethyl group.

16. Compound of formula V; 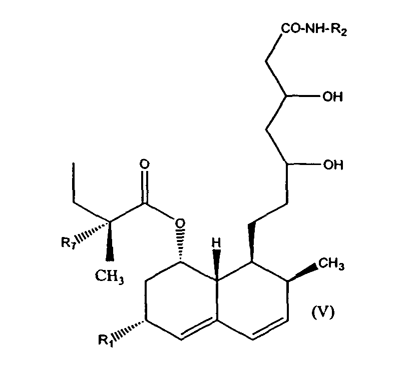 wherein R₂ is a methyl or ethyl group, R₁ is hydrogen or a methyl group and R₇ is a methyl or ethyl group.

17. Compound according to claim 14, wherein said compound is selected from the group consisting of:
lovastatin ethylamide bis-tetrahydropyran-2-ylether;
lovastatin n-butylamide bis-tetrahydropyran-2-ylether;
simvastatin ethylamide bis-tetrahydropyran-2-ylether;
simvastatin n-butylamide bis-tetrahydropyran-2-ylether.

18. Use of the compounds according to claims 14-17 in preparing Simvastatin or a derivative thereof.

19. Compound according to any of the claims 14-17, obtainable by the process according to any of the claims 1 - 13.

## Patentansprüche

1. Verfahren zur Herstellung einer Verbindung der Formel (VI): 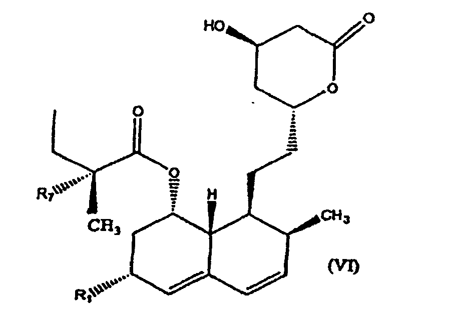 mit den Schritten:
(a) Umsetzen einer Verbindung der Formel (I): 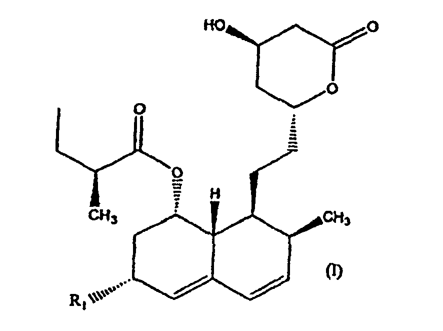 mit einem ringöffnenden Mittel, um eine Verbindung der allgemeinen Formel (II) bereitzustellen: 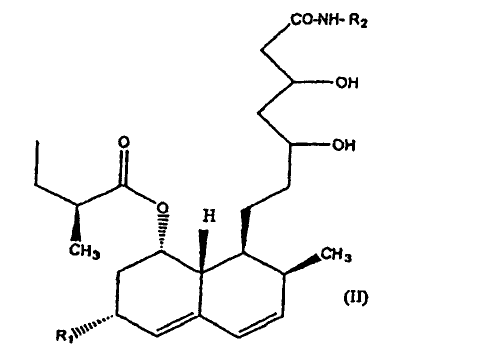 wobei
- R₁ für Wasserstoff oder eine Methylgruppe steht,
- R₂ für eine gerade oder verzweigte Alkylkette mit 1 bis 8 Kohlenstoffatomen, eine Cycloalkylgruppe mit 3 bis 7 Kohlenstoffatomen oder eine Aralkylgruppe, mit 1 bis 6 Kohlenstoffatomen in der Alkylkette, und vorzugsweise für Methyl, Ethyl, Propyl oder Butyl steht;
(b) Schützen der Hydroxygruppen der so gebildeten Verbindung (II) durch Umsetzen der Verbindung der Formel (II) mit einem Schutzmittel, um deren Reaktivität wesentlich zu verlangsamen und eine Verbindung der allgemeinen Formel (III) bereitzustellen: 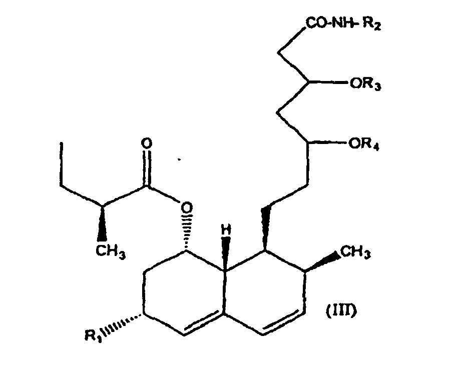 wobei das Schutzmittel die Reaktivität der Verbindung (II) verlangsamt, indem das Wasserstoffatom der Hydroxygruppen davon mit einer kohlenstoff-endständigen Schutzgruppe R₃, R₄, ersetzt wird, wobei R₃ und R₄ jeweils unabhängig für Tetrahydropyran-2-yl, Tetrahydrothiopyran-2-yl, 4-Methoxytetrahydropyran-2-yl, eine Tetrahydrofuranylgruppe oder eine Dioxanylgruppe stehen; und
(c) Umsetzen der Verbindung (III) mit einem Alkylierungsmittel der Formel R₇X, um eine Verbindung der Formel (IV) zu erhalten: 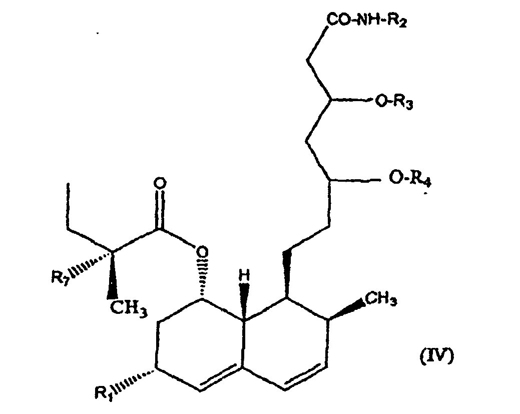 wobei
- R₇ für eine Methyl- oder Ethylgruppe steht, und
- X für eine Abgangsgruppe, vorzugsweise ein Halogenid, am meisten bevorzugt Chlor, Brom oder lod steht;
(d) Hydrolysieren der kohlenstoff-endständigen Schutzgruppen R₃ und R₄ der Verbindung (IV), um eine Verbindung der Formel (V) zu erhalten: 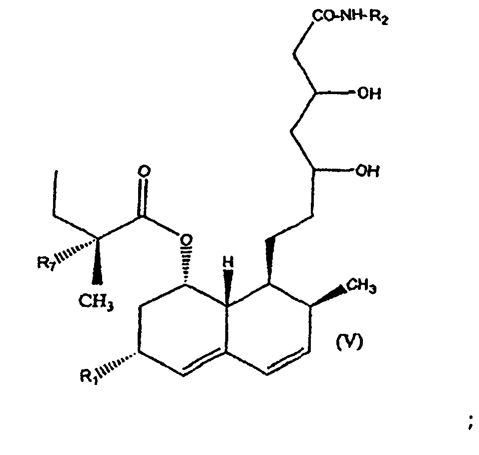 und
(e) Hydrolysieren der Amidgruppierung der Verbindung (V) und Relactonisieren, um die Verbindung (VI) zu erhalten.

2. Verfahren nach Anspruch 1, bei welchem das ringöffnende Mittel ein Amin der allgemeinen Formel R₂NH₂ umfasst, wobei R₂ wie in Anspruch 1 definiert für eine gerade oder verzweigte Alkylkette mit 1 bis 8 Kohlenstoffatomen, eine Cycloalkylgruppe mit 3 bis 7 Kohlenstoffatomen oder eine Aralkylgruppe, mit 1 bis 6 Kohlenstoffatomen in der Alkylkette, und vorzugsweise für Methyl, Ethyl, Propyl oder Butyl steht.

3. Verfahren nach Anspruch 1 oder 2, bei welchem R₃ und R₄ gleich sind.

4. Verfahren zur Herstellung einer Verbindung der Formel (III): 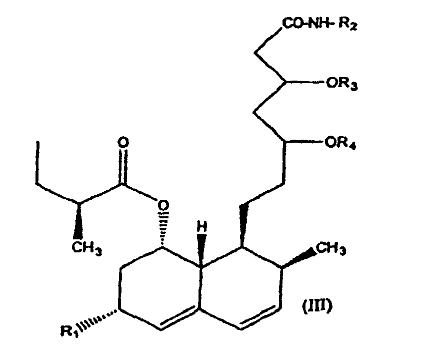 wobei:
- R₁ für Wasserstoff oder eine Methylgruppe steht,
- R₂ für eine gerade oder verzweigte Alkylkette mit 1 bis 8 Kohlenstoffatomen, eine Cycloalkylgruppe mit 3 bis 7 Kohlenstoffatomen oder eine Aralkylgruppe mit 1 bis 6 Kohlenstoffatomen in der Alkylkette, und vorzugsweise für Methyl, Ethyl, Propyl oder Butyl steht,
- R₃ und R₄ jeweils unabhängig für Tetrahydropyran-2-yl, Tetrahydrothiopyran-2-yl, 4-Methoxytetrahydropyran-2-yl, eine Tetrahydrofuranylgruppe oder eine Dioxanylgruppe stehen,
mit den Schritten:
- Umsetzen einer Verbindung der allgemeinen Formel (II): 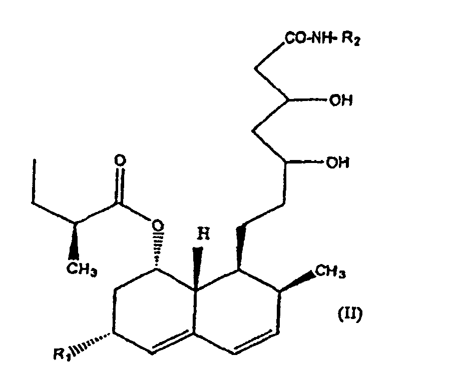 mit einem Schutzmittel, um eine Verbindung der Formel (III) zu bilden, wobei die Wasserstoffatome der Hydroxylgruppen durch kohlenstoffendständige Schutzgruppen R₃ und R₄ ersetzt werden.

5. Verfahren nach Anspruch 4, bei welchem die Verbindung (II) durch Umsetzen einer Verbindung der allgemeinen Formel (I): 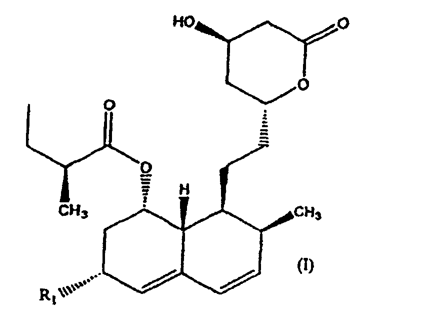 mit einem ringöffnenden Mittel bereitgestellt wird, wobei R₁ wie in Anspruch 4 definiert ist.

6. Verfahren nach Anspruch 5, bei welchem das ringöffnende Mittel ein Amin der allgemeinen Formel R₂NH₂ umfasst, wobei R₂ für eine gerade oder verzweigte Alkylkette mit 1 bis 8 Kohlenstoffatomen, eine Cycloalkylgruppe mit 3 bis 7 Kohlenstoffatomen oder eine Aralkylgruppe, mit 1 bis 6 Kohlenstoffatomen in der Alkylkette steht.

7. Verfahren nach Anspruch 5 oder 6, bei welchem R₃ und R₄ ausgewählt sind aus der Gruppe bestehend aus 1,4-Dioxan-2-yl und Tetrahydrofuran-2-yl.

8. Verfahren nach einem der Ansprüche 5 bis 7, bei welchem das Schutzmittel eine Dihydropyranverbindung ist und R₃ und R₄ beide für die gleiche Tetrahydropyranylgruppe stehen.

9. Verfahren nach einem der Ansprüche 5 bis 8, mit dem weiteren Schritt des Alkylierens der Verbindung (III), um eine Verbindung der Formel (IV) zu erhalten:

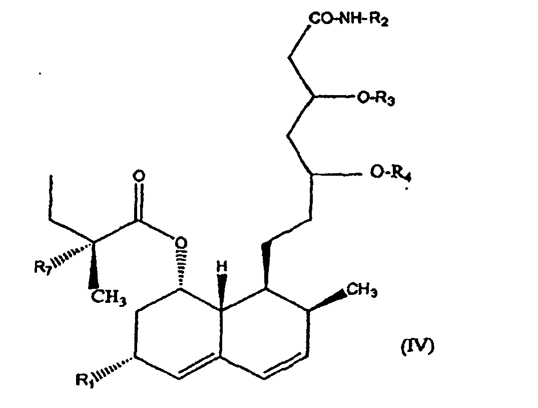

10. Verfahren nach Anspruch 9, bei welchem der Alkylierungsschritt durchgeführt wird, indem man die Verbindung der Formel (III) einer Verbindung der Formel R₇-X aussetzt, wobei R₇ für eine Methyl- oder Ethylgruppe steht und X für eine Abgangsgruppe, vorzugsweise ein Halogenid, am meisten bevorzugt Chlor, Brom oder Iod, steht.

11. Verfahren nach Anspruch 10, mit dem weiteren Schritt des Entfernens der kohlenstoff-endständigen Schutzgruppen von der Verbindung (IV), wodurch man die Verbindung der Formel (V) erhält:

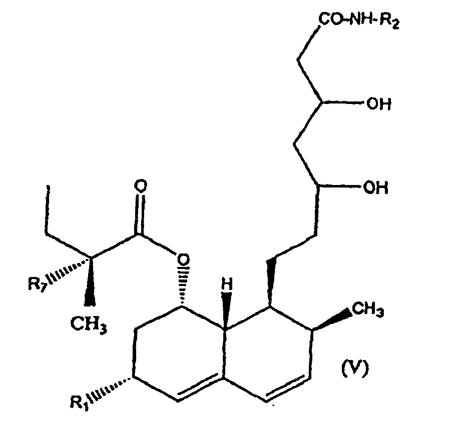

12. Verfahren nach Anspruch 11, bei welchem die Entschützung durch Hydrolysieren der Schutzgruppen erfolgt.

13. Verfahren nach Anspruch 12, mit dem weiteren Schritt der Hydrolyse der Amidgruppierung der Verbindung der Formel (V) und der Relactonisierung, um **dadurch** eine Verbindung der Formel (VI) zu bilden: .

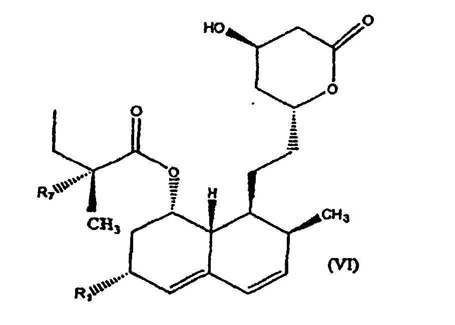

14. Verbindung der Formel (III) oder (IV): 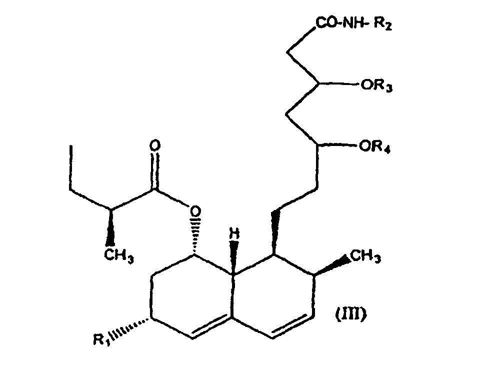 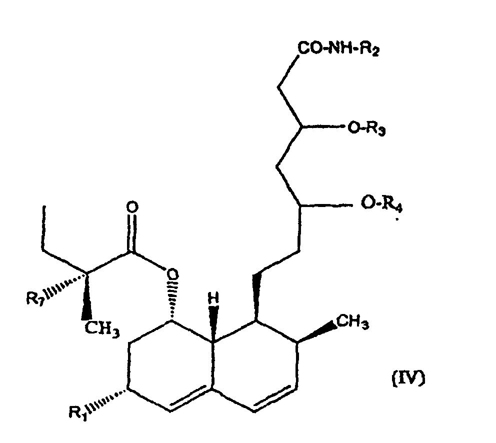 wobei:
- R₁ für Wasserstoff oder Methyl steht;
- R₂ für eine gerade oder verzweigte kettenförmige Alkylgruppe mit 1 bis 8 Kohlenstoffatomen, eine Cycloalkylgruppe mit 3 bis 7 Kohlenstoffatomen oder eine Aralkylgruppe, mit 1 bis 6 Kohlenstoffatomen in der Alkylkette steht;
- R₃ und R₄ jeweils unabhängig für Tetrahydropyran-2-yl, Tetrahydrothiopyran-2-yl, 4-Methoxytetrahydropyran-2-yl, eine Tetrahydrofuranylgruppe oder eine Dioxanylgruppe stehen; und
- R₇ für Methyl oder Ethyl steht.

15. Verbindung der Formel (II): 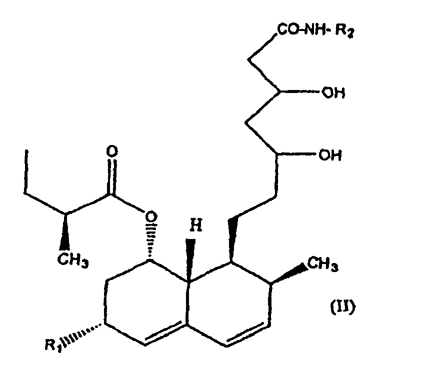 wobei R₁ Wasserstoff oder eine Methylgruppe ist und R₂ eine Methyl- oder Ethylgruppe ist.

16. Verbindung der Formel (V): 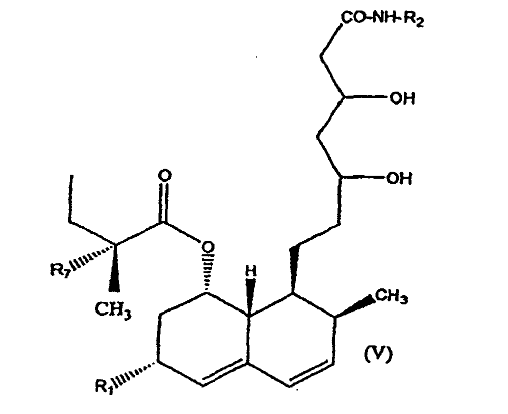 wobei R₂ eine Methyl- oder Ethylgruppe ist, R₁ Wasserstoff oder eine Methylgruppe ist, und R₇ eine Methyl- oder Ethylgruppe ist.

17. Verbindung nach Anspruch 14, bei welcher die Verbindung ausgewählt ist aus der Gruppe bestehend aus:
Lovastatin-ethylamid-bis-tetrahydropyran-2-yl-ether;
Lovastatin-n-butylamid-bis-tetrahydropyran-2-yl-ether;
Simvastatin-ethylamid-bis-tetrahydropyran-2-yl-ether; und
Simvastatin-n-butylamid-bis-tetrahydropyran-2-yl-ether.

18. Verwendung der Verbindungen der Ansprüche 14 bis 17 zur Herstellung von Simvastatin oder einem Derivat davon.

19. Verbindung nach einem der Ansprüche 14 bis 17, erhältlich durch das Verfahren nach einem der Ansprüche 1 bis 13.

## Revendications

1. Procédé de préparation d'un composé de formule VI : 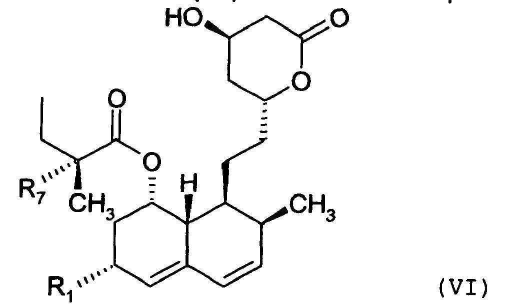 comprenant les étapes consistant à :
(a) faire réagir un composé de formule générale 1 : 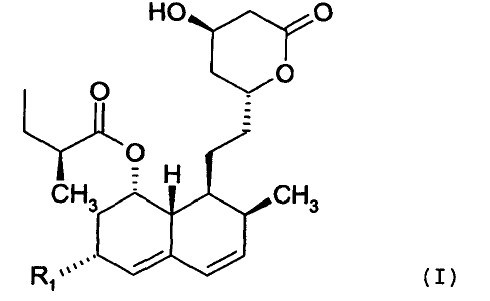 avec un agent d'ouverture de cycle afin de fournir un composé présentant la formule générale II : 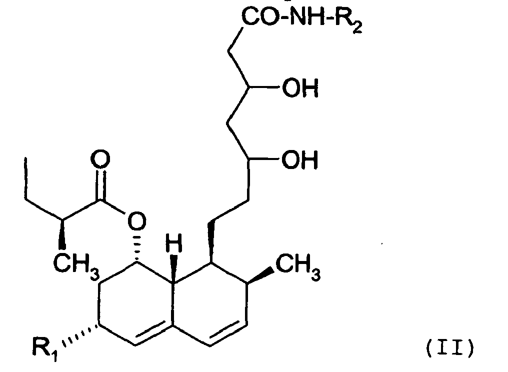 dans laquelle :
- R₁ représente l'hydrogène ou un groupe méthyle,
- R₂ représente une chaîne alkyle linéaire ou ramifiée ayant de 1 à 8 atomes de carbone, un groupe cycloalkyle ayant de 3 à 7 atomes de carbone ou un groupe aralkyle ayant une chaîne alkyle de 1 à 6 atomes de carbone, et représente de préférence le méthyle, l'éthyle, le propyle ou le butyle,
(b) protéger les groupes hydroxy du composé Il ainsi formé en faisant réagir le composé de formule (II) avec un agent protecteur afin de retarder substantiellement sa réactivité et de donner un composé présentant la formule générale III : 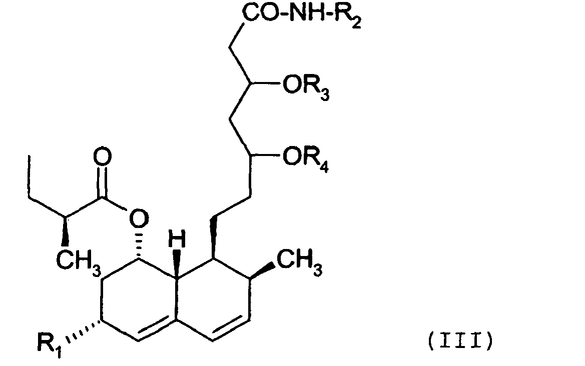 dans laquelle l'agent protecteur retarde la réactivité du composé Il en remplaçant l'atome d'hydrogène des groupes hydroxy de celui-ci par un groupe protecteur à terminaison de carbone, R₃, R₄, où R₃ et R₄ représentent chacun indépendamment un groupe tétrahydropyran-2-yle, tétrahydrothiopyran-2-yle, 4-méthoxytétrahydropyran-2-yle ou tétrahydrofuranyle ou un groupe dioxanyle, et
(c) faire réagir le composé (III) avec un agent d'alkylation de formule R₇X afin d'obtenir un composé présentant la formule (IV) : 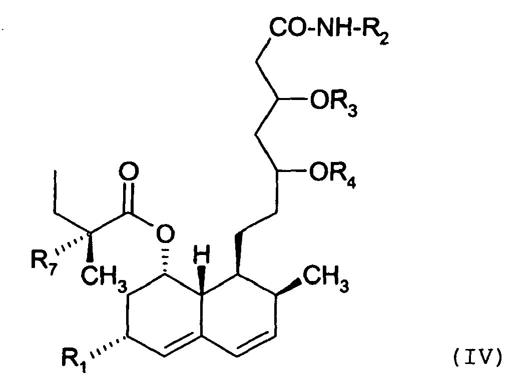 dans laquelle :
- R₇ représente un groupe méthyle ou éthyle et X représente un groupe partant, de préférence, un halogénure, de manière préférée entre toutes, le chlore, le brome ou l'iode,
(d) hydrolyser les groupes protecteurs à terminaison de carbone R₃ et R₄ du composé (IV) afin d'obtenir un composé de formule (V) 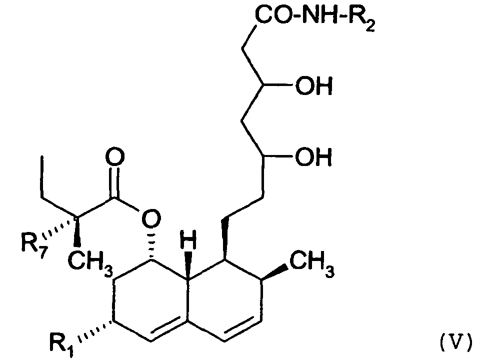 et :
(e) hydrolyser le groupe fonctionnel amide du composé (V) et relactoniser afin de produire le composé (VI).

2. Procédé selon la revendication 1, dans lequel l'agent d'ouverture de cycle comprend une amine présentant la formule générale R₂NH₂ dans laquelle R₂, comme défini dans la revendication 1, représente une chaîne alkyle linéaire ou ramifiée ayant de 1 à 8 atomes de carbone, un groupe cycloalkyle ayant de 3 à 7 atomes de carbone ou un groupe aralkyle ayant une chaîne alkyle de 1 à 6 atomes de carbone, et représente de préférence le méthyle, l'éthyle, le propyle ou le butyle.

3. Procédé selon les revendications 1 ou 2, dans lequel R₃ et R₄ sont identiques.

4. Procédé de préparation d'un composé de formule (III) : 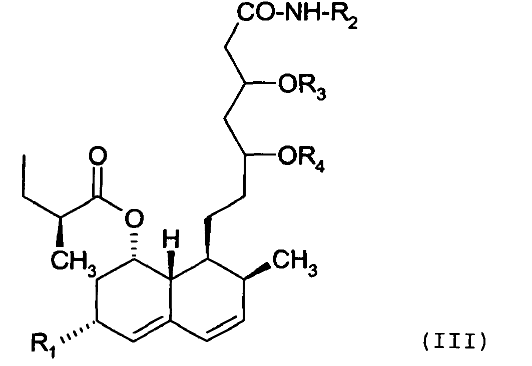 dans laquelle :
- R₁ représente l'hydrogène ou un groupe méthyle,
- R₂ représente une chaîne alkyle linéaire ou ramifiée ayant de 1 à 8 atomes de carbone, un groupe cycloalkyle ayant de 3 à 7 atomes de carbone ou un groupe aralkyle ayant une chaîne alkyle de 1 à 6 atomes de carbone, et représente de préférence le méthyle, l'éthyle, le propyle ou le butyle,
- R₃ et R₄ représentent chacun indépendamment un groupe tétrahydropyran-2-yle, tétrahydrothiopyran-2-yle, 4-méthoxytétrahydropyran-2-yle ou tétrahydrofuranyle ou un groupe dioxanyle,
comprenant l'étape consistant à :
- faire réagir un composé présentant la formule générale (II) : 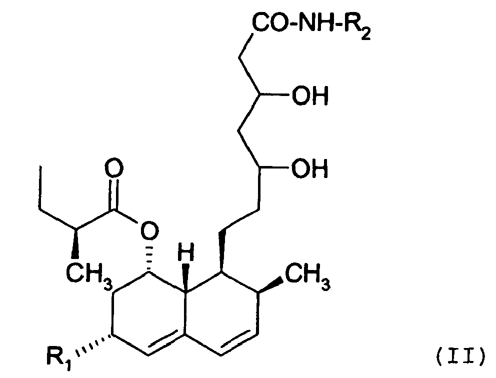 avec un agent protecteur afin de former un composé de formule (III), dans lequel les atomes d'hydrogène des groupes hydroxy sont remplacés par des groupes protecteurs à terminaison de carbone R₃ et R₄.

5. Procédé selon la revendication 4, dans lequel le composé Il est obtenu en faisant réagir un composé de formule générale (I) : 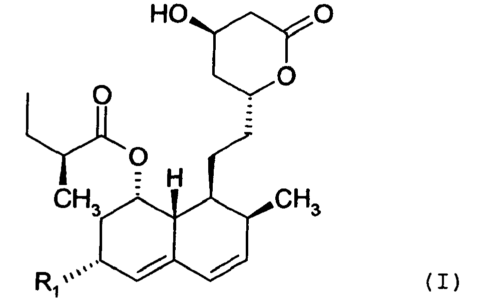 avec un agent d'ouverture de cycle, où R₁ est tel que défini dans la revendication 4.

6. Procédé selon la revendication 5, dans lequel :
l'agent d'ouverture de cycle comprend une amine présentant la formule générale R₂NH₂, dans laquelle R₂ comprend une chaîne alkyle linéaire ou ramifiée ayant de 1 à 8 atomes de carbone, un groupe cycloalkyle ayant de 3 à 7 atomes de carbone ou un groupe aralkyle ayant une chaîne alkyle de 1 à 6 atomes de carbone.

7. Procédé selon l'une quelconque des revendications 5 ou 6, dans lequel R₃ et R₄ sont sélectionnés dans le groupe constitué du 1,4 dioxan-2-yle et du tétrahydrofuran-2-yle.

8. Procédé selon l'une quelconque des revendications 5 à 7 et 10, dans lequel l'agent protecteur est un composé de dihydropyranne et R₃ et R₄ représentent tous les deux le même groupe tétrahydropyranyle.

9. Procédé selon l'une quelconque des revendications 5 à 8, comprenant l'étape supplémentaire d'alkylation du composé (III) afin d'obtenir un composé présentant la formule générale (IV) :

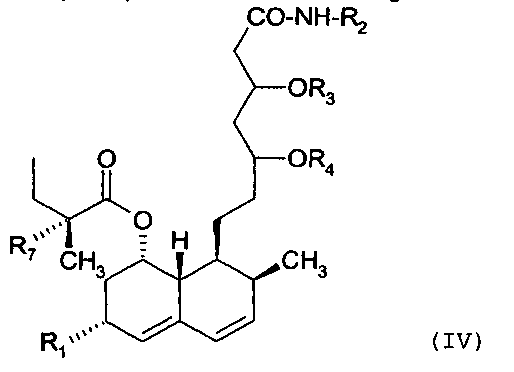

10. Procédé selon la revendication 9, dans lequel ladite étape d'alkylation est mise en oeuvre en soumettant ledit composé de formule (III) à un composé de formule R₇-X, dans laquelle R₇ représente un groupe méthyle ou éthyle et X représente un groupe partant, de préférence un halogénure, et de manière préférée entre toutes, le chlore, le brome ou l'iode.

11. Procédé selon la revendication 10 comprenant l'étape supplémentaire d'élimination des groupes protecteurs à terminaison de carbone du composé (IV), moyennant quoi le composé de formule (V) : 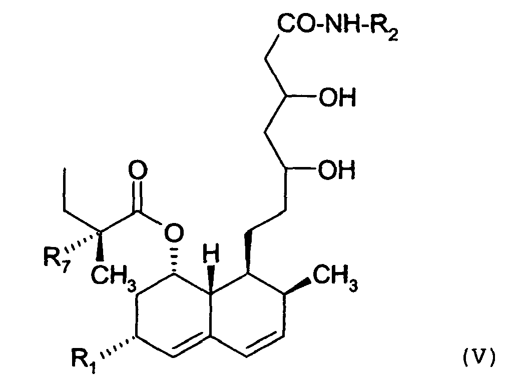 est obtenu.

12. Procédé selon la revendication 11, dans lequel la déprotection est opérée en hydrolysant les groupes protecteurs.

13. Procédé selon la revendication 12, comprenant les étapes supplémentaires d'hydrolyse du groupe fonctionnel amide du composé de formule (V) et de relactonisation afin de former ainsi un composé présentant la formule (VI).

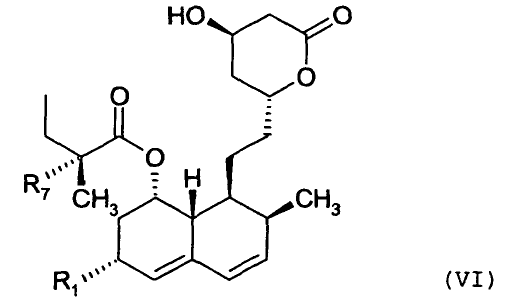

14. Le composé de formule (III) ou (IV), 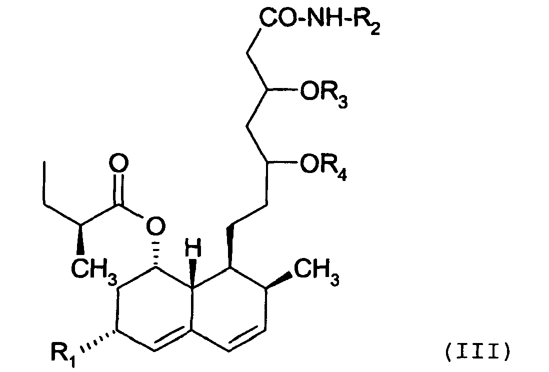 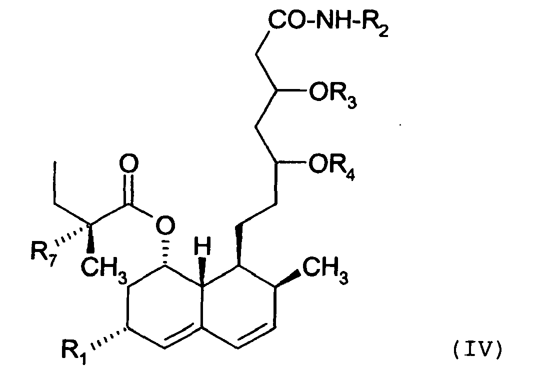 dans laquelle :
- R₁ représente l'hydrogène ou un groupe méthyle,
- R₂ représente un groupe alkyle à chaîne linéaire ou ramifiée ayant de 1 à 8 atomes de carbone, un groupe cycloalkyle ayant de 3 à 7 atomes de carbone ou un groupe aralkyle ayant une chaîne alkyle de 1 à 6 atomes de carbone ;
- R₃ et R₄ représentent chacun indépendamment un groupe tétrahydropyran-2-yle, tétrahydrothiopyran-2-yle, 4-méthoxytétrahydropyran-2-yle ou tétrahydrofuranyle ou un groupe dioxanyle,
- et R₇ représente le méthyle ou l'éthyle.

15. Le composé de formule II : 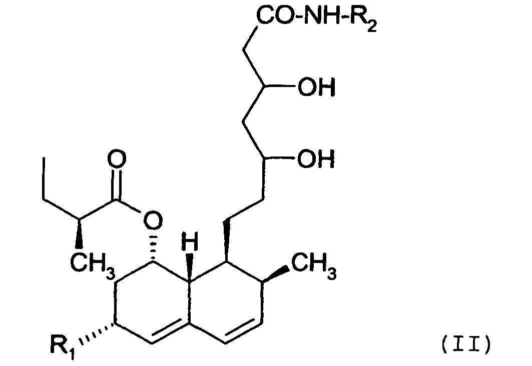 dans laquelle R₁ est l'hydrogène ou un groupe méthyle et R₂ est un groupe méthyle ou éthyle.

16. Le composé de formule V : 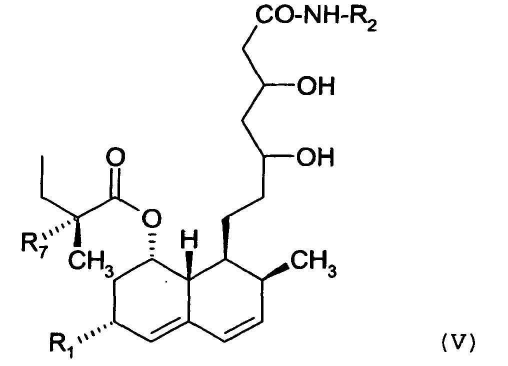 dans laquelle R₂ est un groupe méthyle ou éthyle, R₁ est l'hydrogène ou un groupe méthyle et R₇ est un groupe méthyle ou éthyle.

17. Le composé selon la revendication 16, dans lequel ledit composé est sélectionné dans le groupe constitué de :
lovastatine éthylamide bis-tétrahydropyran-2-yléther ;
lovastatine n-butylamide bis-tétrahydropyran-2-yléther ;
simvastatine éthylamide bis-tétrahydropyran-2-yléther ;
simvastatine n-butylamide bis-tétrahydropyran-2-yléther.

18. Utilisation des composés selon les revendications 14 à 17 pour préparer la simvastatine ou un dérivé de celle-ci.

19. Composé selon l'une quelconque des revendications 14 à 17, susceptible d'être obtenu par le procédé selon l'une quelconque des revendications 1 à 13.
